(19) Europäisches Patentamt
European Patent Office
Office européen des brevets

(11) EP 4 203 907 B1

(12) EUROPEAN PATENT SPECIFICATION

(45) Date of publication and mention of the grant of the patent:
**04.09.2024 Bulletin 2024/36**

(21) Application number: **21770322.2**

(22) Date of filing: **13.08.2021**

(51) International Patent Classification (IPC):
***A61K 8/37*** (2006.01) ***A61K 8/60*** (2006.01)
***A61K 8/67*** (2006.01) ***A61Q 19/00*** (2006.01)
***A61Q 19/08*** (2006.01)

(52) Cooperative Patent Classification (CPC):
**A61K 8/602; A61K 8/375; A61K 8/675; A61Q 19/00; A61Q 19/08**

(86) International application number:
**PCT/JP2021/030482**

(87) International publication number:
**WO 2022/044974 (03.03.2022 Gazette 2022/09)**

(54) **STABLE COMPOSITION COMPRISING POLYDATIN**

STABILE ZUSAMMENSETZUNG MIT POLYDATIN

COMPOSITION STABLE COMPRENANT DE LA POLYDATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2020 JP 2020141567**
**06.10.2020 FR 2010177**

(43) Date of publication of application:
**05.07.2023 Bulletin 2023/27**

(73) Proprietors:
- **L'OREAL**
  **75008 Paris (FR)**
  Designated Contracting States:
  **AT BE BG CH CY CZ DE DK EE ES FI FR GB GR HR HU IE IS IT LI LT LU LV MC MK MT NL NO PL PT RO RS SE SI SK SM TR**
- **Iima, Yusuke**
  **Kawasaki-shi, Kanagawa 213-0012 (JP)**
  Designated Contracting States:
  **AL**
- **Suzuki, Jun**
  **Kawasaki-shi, Kanagawa 213-0012 (JP)**
  Designated Contracting States:
  **AL**
- **Bodnar, Brian**
  **Clark, NJ (US)**
  Designated Contracting States:
  **AL**

(72) Inventors:
- **IIMA, Yusuke**
  **Kawasaki-shi, Kanagawa 2130012 (JP)**
- **SUZUKI, Jun**
  **Kawasaki-shi, Kanagawa 2130012 (JP)**
- **BODNAR, Brian**
  **Clark, NJ 07066 (US)**

(74) Representative: **Casalonga**
**Casalonga & Partners**
**Bayerstraße 71/73**
**80335 München (DE)**

(56) References cited:
**EP-A1- 1 378 491**
**FR-A1- 2 734 479**
**US-A1- 2017 281 505**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 203 907 B1

## Description

TECHNICAL FIELD

The present invention relates to a composition, preferably a cosmetic or dermatological composition, which includes polydatin but is stable against, in particular, light.

BACKGROUND ART

The formation of free radicals is widely considered to play a significant role in the mechanisms of skin aging. Free radicals are highly reactive molecular species with unpaired electrons that can directly damage various cellular membranes, lipids, proteins, RNA and DNA. The damaging effects of said free radicals, often present in the form of reactive oxygen species, are induced inside the tissue and cells during normal metabolism and externally through various oxidative stresses. UV exposure and environmental pollution can accelerate skin aging by producing free radicals in skin. Antioxidants protect cells from the damage of oxidative stress by scavenging free radicals and inhibiting oxidation reactions. The topical application of antioxidants is broadly used in skin care products to prevent skin aging. It has been previously shown in the field of cosmetics that polyphenols act synergistically with other antioxidants such as Vitamin E and carotenoids.

DISCLOSURE OF INVENTION

Polydatin (also known as piceid) is an antioxidant, and may be used as a potent whitening active ingredient. However, it is difficult for a composition including polydatin to be stable against, in particular, light.

An objective of the present invention is to provide a composition which includes polydatin, but is stable against, in particular, light.

The above objective of the present invention can be achieved with a composition comprising:

(a) polydatin;
(b) at least one caffeoyl quinic acid compound;
(c) at least one Vitamin B3 compound; and
(d) water,

wherein
the amount of the (b) caffeoyl quinic acid compound is 0.5% by weight or more, preferably 1.0% by weight or more, and more preferably 1.5% by weight or more, relative to the total weight of the composition.

The (a) polydatin may be derived from plants.

The amount of the (a) polydatin in the composition according to the present invention may range from 0.01% to 3% by weight, preferably from 0.05% to 2% by weight, and more preferably from 0.1% to 1% by weight, relative to the total weight of the composition.

The (b) caffeoyl quinic acid compound may be represented by the following formula (I):

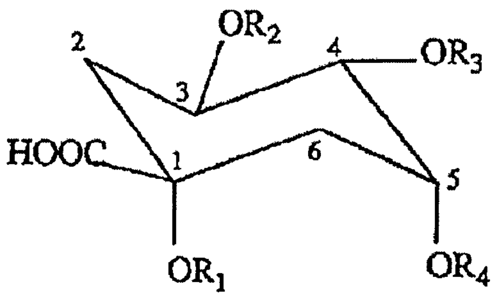

(I)

in which $R_1$, $R_2$, $R_3$, and $R_4$ independently represents a hydrogen atom or the caffeoyl radical represented by the following formula (II):

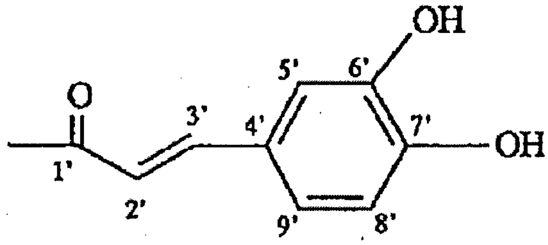

(II)

provided that at least one of $R_1$, $R_2$, $R_3$, and $R_4$ represents the caffeoyl radical of the formula (II).

The (b) caffeoyl quinic acid compound may be chlorogenic acid.

The (b) caffeoyl quinic acid compound may be derived from plants.

The amount of the (b) caffeoyl quinic acid compound(s) in the composition according to the present invention may range from 0.5% to 15% by weight, preferably from 1.0% to 10% by weight, and more preferably from 1.5% to 5% by weight, relative to the total weight of the composition.

The (c) Vitamin B3 compound may be represented by the following formula (III):

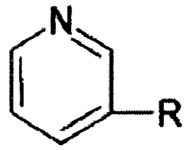

(III)

in which R denotes $^{-}CONH_2$, -COOH, $CH_2OH$, $-CO-NH-CH_2-COOH$ or -CO-NH-OH.

The (c) Vitamin B3 compound may be niacinamide.

The amount of the (c) Vitamin B3 compound(s) in the composition according to the present invention may range from 0.1% to 15% by weight, preferably from 0.5% to 10% by weight, and more preferably from 1% to 5% by weight, relative to the total weight of the composition.

The weight ratio of the amount of the (a) polydatin/the (b) caffeoyl quinic acid compound(s) may be from 0.1 to 1, preferably from 0.2 to 0.5, and more preferably from 0.3 to 0.4.

The weight ratio of the amount of the (a) polydatin/the (c) Vitamin B3 compound(s) may be from 0.01 to 0.5, preferably from 0.05 to 0.3, and more preferably from 0.1 to 0.2.

The weight ratio of the amount of the (c) Vitamin B3 compound(s)/the (b) caffeoyl quinic acid compound(s) may be from 1 to 8, preferably from 1.5 to 4, and more preferably from 2 to 3.

The composition according to the present invention may be in the form of an OAV emulsion, preferably a nano- or micro- O/W emulsion, and more preferably a nano- or micro- O/W gel emulsion.

The present invention also relates to a cosmetic process for treating a keratin substance such as skin, comprising the step of applying the composition according to the present invention to the keratin substance.

BEST MODE FOR CARRYING OUT THE INVENTION

After diligent research, the inventors have discovered that it is possible to provide a composition which includes polydatin but is stable against, in particular, light.

Thus, the composition according to the present invention comprises

(a) polydatin;
(b) at least one caffeoyl quinic acid compound;
(c) at least one Vitamin B3 compound; and
(d) water,

wherein
the amount of the (b) caffeoyl quinic acid compound is 0.5% by weight or more, preferably 1.0% by weight or more, and more preferably 1.5% by weight or more, relative to the total weight of the composition.

The composition according to the present invention is stable against several factors, including cooling and light, in particular light such as visible light, UV rays and IR rays. Thus, the composition according to the present invention can have superior thermal stability and photostability, in particular photostability.

The term "superior thermal stability" here means that, after cooling to, for example, 4°C, for a certain period of time such as 1 month, the composition according to the present invention is visually homogeneous and does not cause precipitation of polydatin which may be in the form of crystal particles.

The term "superior photostability" here means that 50% or more, preferably 55% or more, and more preferably 60% or more of polydatin before being exposed to light can remain after being exposed to light.

Hereinafter, the composition according to the present invention will be explained in a more detailed manner.

[Polydatin]

The composition according to the present invention comprises (a) polydatin.

Polydatin, which may be described as 3,4',5-trihydroxystibene-3-β-mono-D glucoside, is a stilbenoid. Stilbenoids have strong natural antioxidant properties of free-radical quenching and limitation of oxidative stress. These properties can be used for skincare or suncare products, including compositions according to the present invention. When exposed to sun light or other radiation, polydatin, which is active in a trans-conformation, photoisomerizes into cis-polydatin which has less biological activity.

The amount of the (a) polydatin in the composition according to the present invention may be 0.01% by weight or more, preferably 0.05% by weight or more, and more preferably 0.1% by weight or more, relative to the total weight of the composition.

The amount of the (a) polydatin in the composition according to the present invention may be 3% by weight or less, preferably 2% by weight or less, and more preferably 1% by weight or less, relative to the total weight of the composition, with the proviso that the amount of the (a) polydatin is not zero.

The amount of the (a) polydatin in the composition according to the present invention may range from 0.01% to 3% by weight, preferably from 0.05% to 2% by weight, and more preferably from 0.1% to 1% by weight, relative to the total weight of the composition.

[Caffeoyl Quinic Acid Compound]

The composition according to the present invention comprises (b) at least one caffeoyl quinic acid compound. If two or more caffeoyl quinic acid compounds are used, they may be the same or different.

The (b) caffeoyl quinic acid compound here means a compound which has a quinic acid structure with at least one caffeoyl radical or group.

The (b) caffeoyl quinic acid compound may be represented by the following formula (I):

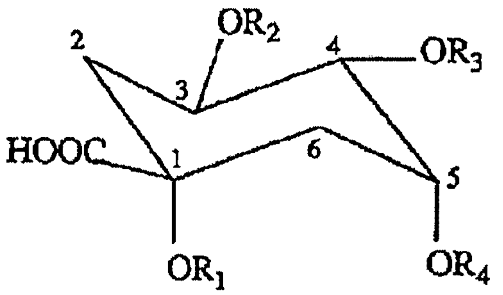

(I)

in which $R_1$, $R_2$, $R_3$, and $R_4$ independently represents a hydrogen atom or the caffeoyl radical represented by the following formula (II):

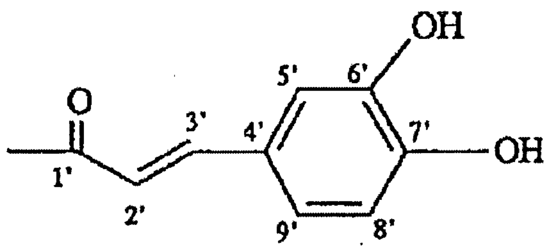

(II)

provided that at least one of $R_1$, $R_2$, $R_3$, and $R_4$ represents the caffeoyl radical of the formula (II).

As examples of the (b) caffeoyl quinic acid compound, mention may be made of 3-caffeoylquinic acid (chlorogenic acid), 4,5-di-caffeoylquinic acid, 3,5-di-caffeoylquinic acid, 1,3-di-caffeoylquinic acid, 3,4-di-caffeoylquinic acid, 3,4,5-tri-caffeoylquinic acid, and mixtures thereof.

It is preferable that the (b) caffeoyl quinic acid compound be chlorogenic acid.

The amount of the (b) caffeoyl quinic acid compound(s) in the composition according to the present invention is 0.5% by weight or more, and may preferably be 1.0% by weight or more, and more preferably 1.5% by weight or more, relative to the total weight of the composition.

The amount of the (b) caffeoyl quinic acid compound(s) in the composition according to the present invention may be 15% by weight or less, preferably 10% by weight or less, and more preferably 5% by weight or less, relative to the total weight of the composition, with the proviso that the amount of the (b) caffeoyl quinic acid compound is not zero.

The amount of the (b) caffeoyl quinic acid compound(s) in the composition according to the present invention may range from 0.5% to 15% by weight, preferably from 1.0% to 10% by weight, and more preferably from 1.5% to 5% by weight, relative to the total weight of the composition.

[Vitamin B3 Compound]

The composition according to the present invention comprises (c) at least one Vitamin B3 compound. If two or more Vitamin B3 compounds are used, they may be the same or different.

The Vitamin B3 compound here means the compound in the group of Vitamin B3 as well as derivatives thereof.

Vitamin B3, also called vitamin PP, here is a compound represented by the following formula (III):

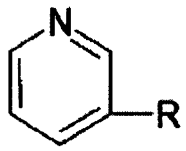

(III)

in which R may be $-CONH_2$ (niacinamide), -COOH (nicotinic acid or niacin), or $CH_2OH$ (nicotinyl alcohol), $-CO-NH-CH_2-COOH$ (nicotinuric acid) or -CO-NH-OH (niconityl hydroxamic acid). Niacinamide is preferable.

Vitamin B3 derivatives that may be mentioned include, for example, nicotinic acid esters such as tocopherol nicotinate, amides derived from niacinamide by substitution of the hydrogen groups of $-CONH_2$, products from reaction with carboxylic acids and amino acids, esters of nicotinyl alcohol and of carboxylic acids such as acetic acid, salicyclic acid, glycolid acid or palmitic acid.

Mention may also be made of the following derivatives: 2-chloronicotinamide, 6-methylnicotinamide, 6-aminonicotinamide, N-methylnicotinamide, N,N-dimethylnicotinamide, N,N-diethylnicotinamide, N-picolylnicotinamide, N-allylnicotinamide, N-(hydroxymethyl)nicotinamide, quinolinic acid imide, nicotinanilide, N-benzylnicotinamide, N-ethylnicotinamide, nifenazone, nicotinaldehyde, isonicotinic acid, methylisonicotinic acid, thionicotinamide, nialamide, 2-mercaptonicotinic acid, nicomol and niaprazine, methyl nicotinate and sodium nicotinate.

Other vitamin B3 derivatives that may also be mentioned include its inorganic salts, such as chlorides, bromides, iodides or carbonates, and its organic salts, such as the salts obtained by reaction with carboxylic acids, such as acetate, salicylate, glycolate, lactate, malate, citrate, mandelate, tartrate, etc.

The amount of the (c) Vitamin B3 compound(s) in the composition according to the present invention may be 0.1% by weight or more, preferably 0.5% by weight or more, and more preferably 1% by weight or more, relative to the total weight of the composition.

The amount of the (c) Vitamin B3 compound(s) in the composition according to the present invention may be 15% by weight or less, preferably 10% by weight or less, and more preferably 5% by weight or less, relative to the total weight of the composition, with the proviso that the amount of the (c) Vitamin B3 compound is not zero.

The amount of the (c) Vitamin B3 compound(s) in the composition according to the present invention may range from 0.1% to 15% by weight, preferably from 0.5% to 10% by weight, and more preferably from 1% to 5% by weight, relative to the total weight of the composition.

(Weight Ratio)

The weight ratio of the amount of the (a) polydatin/the (b) caffeoyl quinic acid compound(s) may be 0.1 or more, preferably 0.2 or more, and more preferably 0.3 or more.

The weight ratio of the amount of the (a) polydatin/the (b) caffeoyl quinic acid compound(s) may be 1 or less, preferably 0.5 or less, and more preferably 0.4 or less.

The weight ratio of the amount of the (a) polydatin/the (b) caffeoyl quinic acid compound(s) may be from 0.1 to 1, preferably from 0.2 to 0.5, and more preferably from 0.3 to 0.4.

The weight ratio of the amount of the (a) polydatin/the (c) Vitamin B3 compound(s) may be 0.01 or more, preferably 0.05 or more, and more preferably 0.1 or more.

The weight ratio of the amount of the (a) polydatin/the (c) Vitamin B3 compound(s) may be 0.5 or less, preferably 0.3 or less, and more preferably 0.2 or less.

The weight ratio of the amount of the (a) polydatin/the (c) Vitamin B3 compound(s) may be from 0.01 to 0.5, preferably from 0.05 to 0.3, and more preferably from 0.1 to 0.2.

The weight ratio of the amount of the (c) Vitamin B3 compound(s)/the (b) caffeoyl quinic acid compound(s) may be 1 or more, preferably 1.5 or more, and more preferably 2 or more.

The weight ratio of the amount of the (c) Vitamin B3 compound(s)/the (b) caffeoyl quinic acid compound(s) may be 8 or less, preferably 4 or less, and more preferably 3 or less.

The weight ratio of the amount of the (c) Vitamin B3 compound(s)/the (b) caffeoyl quinic acid compound(s) may be from 1 to 8, preferably from 1.5 to 4, and more preferably from 2 to 3.

[Water]

The composition according to the present invention comprises (d) water.

The amount of the (d) water in the composition according to the present invention may be 50% by weight or more, preferably 60% by weight or more, and more preferably 70% by weight or more, relative to the total weight of the composition.

On the other hand, the amount of the (d) water in the composition according to the present invention may be 95% by weight or less, preferably 90% by weight or less, and more preferably 85% by weight or less, relative to the total weight of the composition, with the proviso that the amount of the (d) water is not zero.

The amount of (d) water in the composition according to the present invention may range from 50% to 95% by weight, preferably from 60% to 90% by weight, more preferably from 70% to 85% by weight, relative to the total weight of the composition.

[Solubilizer]

The composition according to the present invention may further comprise at least one solubilizer. If two or more solubilizers are used, they may be the same or different.

The solubilizer can solubilize the (a) polydatin furthermore in the (d) water.

One or more solubilizers may be a hydrotrope, but the solubilizers are not required to be hydrotropes. Hydrotropes (or hydrotropic agents) are a diverse class of water-soluble compounds that are characterized by an amphiphilic molecular structure and an ability to dramatically increase the solubility of poorly soluble organic molecules in water.

Many hydrotropes have an aromatic structure with an ionic moiety, while some of them are linear alkyl chains. Although hydrotropes noticeably resemble surfactants and have the ability to reduce surface tension, their small hydrophobic units and relatively shorter alkyl chain distinguish them as a separate class of amphiphiles. Consequently their hydrophobicity is not sufficient enough to create well organized self-associated structures, such as micelles, even with a high concentration.

Common hydrotropic molecules include: sodium 1,3-benzenedisulfonate, sodium benzoate, sodium 4-pyridinecarboxylate, sodium salicylate, sodium benzene sulfonate, caffeine, sodium p-toluene sulfonate, sodium butyl monoglycolsulfate, 4-aminobenzoic acid HCl, sodium cumene sulfonate, 2-methacryloyloxyethyl phosphorylcholine, resorcinol, butylurea, pyrogallol, N-picolylacetamide 3.5, procaine HCl, proline HCl, pyridine, 3-picolylamine, sodium ibuprofen, sodium xylenesulfonate, ethyl carbamate, pyridoxal hydrochloride, sodium benzoate, 2-pyrrolidone, ethylurea, N,N-dimethylacetamide, N-methylacetamide, and isoniazid. Hydrotropes can be found in Lee J. et al., "Hydrotropic Solubilization of Paclitaxel: Analysis of Chemical Structures for Hydrotropic Property", Pharmaceutical Research, Vol. 20, No. 7, 2003; and Lee S. et al., "Hydrotropic Polymers: Synthesis and Characterization of Polymers Containing Picolylnicotinamide Moieties", Macromolecules, 36, 2248-2255, 2003. Preferable hydrotropes may include caffeine, sodium PCA, sodium salicylate, urea, and dihydroxyethyl urea.

The amount of the solubilizer in the composition according to the present invention may be 0.01% by weight or more, preferably 0.05% by weight or more, and more preferably 0.1% by weight or more, relative to the total weight of the composition.

On the other hand, the amount of the solubilizer in the composition according to the present invention may be 4% by weight or less, preferably 3% by weight or less, and more preferably 2% by weight or less, relative to the total weight of the composition, with the proviso that the amount of the solubilizer is not zero.

The amount of the solubilizer in the composition according to the present invention may range from 0.01% to

4% by weight, preferably from 0.05% to 3% by weight, more preferably from 0.1% to 2% by weight, relative to the total weight of the composition.

[Oil]

The composition according to the present invention may further comprise at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The oil may be a non-polar oil such as a hydrocarbon oil, a silicone oil, or the like; a polar oil such as a plant or animal oil and an ester oil or an ether oil; or a mixture thereof.

The oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched $C_1$-$C_{26}$ aliphatic monoacids or polyacids and of saturated or unsaturated, linear or branched $C_1$-$C_{26}$ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of $C_4$-$C_{22}$ dicarboxylic or tricarboxylic acids and of $C_1$-$C_{22}$ alcohols, and esters of monocarboxylic, dicarboxylic, or tricarboxylic acids and of non-sugar $C_4$-$C_{26}$ dihydroxy, trihydroxy, tetrahydroxy, or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of $C_6$-$C_{30}$ and preferably $C_{12}$-$C_{22}$ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated $C_6$-$C_{30}$ and preferably $C_{12}$-$C_{22}$ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate® DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:

(i) cyclic polydialkylsiloxanes comprising from 3 to 7 and preferably 4 to 5 silicon atoms. These are, for example, octamethylcyclotetrasiloxane sold in particular under the name Volatile Silicone® 7207 by Union Carbide or Silbione® 70045 V2 by Rhodia, decamethylcyclopentasiloxane sold under the name Volatile Silicone® 7158 by Union Carbide, Silbione® 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile® FZ 3109 sold by the company Union Carbide, of the formula:

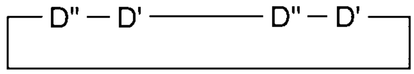

with D'': 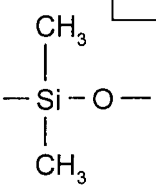 and with D': 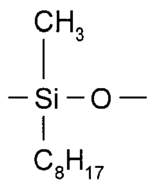

Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and

(ii) linear volatile polydialkylsiloxanes containing 2 to 9 silicon atoms and having a viscosity of less than or equal to $5 \times 10^{-6}$ $m^2/s$ at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics. The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:

- the Silbione® oils of the 47 and 70 047 series or the Mirasil® oils sold by Rhodia, for instance the oil 70 047 V 500 000;
- the oils of the Mirasil® series sold by the company Rhodia;
- the oils of the 200 series from the company Dow Coming, such as DC200 with a viscosity of 60 000 $mm^2/s$; and
- the Viscasil® oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.
Among the silicones containing aryl groups, mention may be made of polydiarylsiloxanes, especially polydiphenylsiloxanes and polyalkylarylsiloxanes such as phenyl silicone oil.
The phenyl silicone oil may be chosen from the phenyl silicones of the following formula:

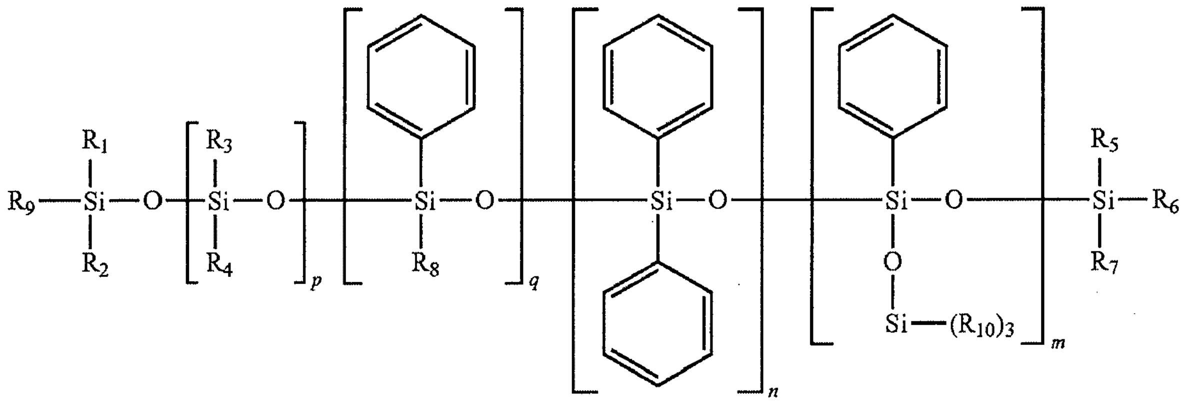

in which

$R_1$ to $R_{10}$, independently of each other, are saturated or unsaturated, linear, cyclic or branched $C_1$-$C_{30}$ hydrocarbon-based radicals, preferably $C_1$-$C_{12}$ hydrocarbon-based radicals, and more preferably $C_1$-$C_6$ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:

- the Silbione® oils of the 70 641 series from Rhodia;
- the oils of the Rhodorsil® 70 633 and 763 series from Rhodia;
- the oil Dow Coming 556 Cosmetic Grade Fluid from Dow Coming;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (Ri to $R_{10}$ are methyl; p, q, and n = 0; m=1 in the above formula) is preferable.
The organomodified liquid silicones may especially contain polyethyleneoxy and/or polypropyleneoxy groups. Mention may thus be made of the silicone KF-6017 proposed by Shin-Etsu, and the oils Silwet® L722 and L77 from the company Union Carbide.
The hydrocarbon oils may be chosen from:

- linear or branched, optionally cyclic, $C_6$-$C_{16}$ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam®, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.
The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.
The fatty alcohol may have the structure R-OH wherein R is chosen from saturated and unsaturated, linear

and branched radicals containing from 4 to 40 carbon atoms, preferably from 6 to 30 carbon atoms, and more preferably from 12 to 20 carbon atoms. In at least one embodiment, R may be chosen from $C_{12}$-$C_{20}$ alkyl and $C_{12}$-$C_{20}$ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated $C_6$-$C_{30}$ alcohols, preferably straight or branched, saturated $C_6$-$C_{30}$ alcohols, and more preferably straight or branched, saturated $C_{12}$-$C_{20}$ alcohols.

The term "saturated fatty alcohol" here means an alcohol having a long aliphatic saturated carbon chain. It is preferable that the saturated fatty alcohol be selected from any linear or branched, saturated $C_6$-$C_{30}$ fatty alcohols. Among the linear or branched, saturated $C_6$-$C_{30}$ fatty alcohols, linear or branched, saturated $C_{12}$-$C_{20}$ fatty alcohols may preferably be used. Any linear or branched, saturated $C_{16}$-$C_{20}$ fatty alcohols may be more preferably used. Branched $C_{16}$-$C_{20}$ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from cetyl alcohol, octyldodecanol, hexyldecanol, and mixtures thereof.

It is also preferable that the oil be chosen from oils with a molecular weight below 600 g/mol.

Preferably, the oil has a low molecular weight such as below 600 g/mol, chosen among ester oils with a short hydrocarbon chain or chains ($C_1$-$C_{12}$) (e.g., isopropyl lauroyl sarcosinate, isopropyl myristate, isopropyl palmitate, isononyl isononanoate, and ethyl hexyl palmitate), silicone oils (e.g., volatile silicones such as cyclohexasiloxane), hydrocarbon oils (e.g., isododecane, isohexadecane, and squalane), branched and/or unsaturated fatty alcohol ($C_{12}$-$C_{30}$) type oils such as octyldodecanol and oleyl alcohol, and ether oils such as dicaprylyl ether.

It is preferable that the oil be chosen from polar oils, and more preferably from ester oils.

The amount of the oil(s) in the composition according to the present invention may be 0.01% by weight or more, preferably 0.05% by weight or more, and more preferably 0.1% by weight or more, relative to the total weight of the composition.

The amount of the oil(s) in the composition according to the present invention may be 10% by weight or less, preferably 5% by weight or less, and more preferably 1% by weight or less, relative to the total weight of the composition, with the proviso that the amount of the oil is not zero.

The amount of the oil(s) in the composition according to the present invention may be from 0.01% to 10% by weight, preferably from 0.05% to 5% by weight, and more preferably from 0.1% to 1% by weight, relative to the total weight of the composition.

[Emulsifier]

The composition according to the present invention may further comprise at least one emulsifier. If two or more emulsifiers are used, they may be the same or different.

The emulsifiers may be surfactants. The surfactants may be selected from cationic, anionic, amphoteric and nonionic surfactants. It may be preferable that the composition according to the present invention includes at least one anionic surfactant and at least one nonionic surfactant.

It may be preferable that the anionic surfactant be selected from amino acid type surfactants.

Non-limiting examples of amino acid type surfactants include potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, sodium methyl stearoyl taurate, dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, sodium undecylenoyl glutamate, cocoyl methyl β-alanine, lauroyl β-alanine, lauroyl methyl β-alanine, myristoyl β-alanine, potassium lauroyl methyl β-alanine, sodium cocoyl alaninate, sodium cocoyl methyl β-alanine and sodium myristoyl methyl β-alanine palmitoyl glycine, sodium lauroyl glycine, sodium cocoyl glycine, sodium myristoyl glycine, potassium lauroyl glycine, potassium cocoyl glycine, potassium lauroyl

sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof.

Mention is also made of potassium lauroyl sarcosinate, potassium cocoyl sarcosinate, sodium cocoyl sarcosinate, sodium lauroyl sarcosinate, sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, dipotassium caproyl aspartate, and mixtures thereof.

The amount of the amino acid type surfactant(s) in the composition according to the present invention may be 0.001% by weight or more, preferably 0.005% by weight or more, and more preferably 0.01% by weight or more, relative to the total weight of the composition.

On the other hand, the amount of the amino acid type surfactant(s) in the composition according to the present invention may be 1% by weight or less, preferably 0.5% by weight or less, and more preferably 0.1% by weight or less, relative to the total weight of the composition, with the proviso that the amount of the amino acid type surfactant is not zero.

The amount of the amino acid type surfactant in the composition according to the present invention may range from 0.001% to 1% by weight, preferably from 0.005% to 0.5% by weight, more preferably from 0.01% to 0.1% by weight, relative to the total weight of the composition.

It may be preferable that the composition according to the present invention includes at least one nonionic surfactant, more preferably at least one polyglyceryl fatty acid ester, and even more preferably a combination of first and second polyglyceryl fatty acid esters.

(First Polyglyceryl Fatty Acid Ester)

The composition according to the present invention may comprise at least one first polyglyceryl fatty acid ester having an HLB value of 12.0 or more, preferably 12.5 or more, and more preferably 13.0 or more. A single type of first polyglyceryl fatty acid ester may be used, but two or more different types of first polyglyceryl fatty acid ester may be used in combination.

The first polyglyceryl fatty acid ester can function as a surfactant, in particular a nonionic surfactant.

The first polyglyceryl fatty acid may have an HLB value of 12.0 to 17.0, preferably 12.5 to 16.0, and more preferably 13.5 to 15.0.

The term HLB ("hydrophilic-lipophilic balance") is well known to those skilled in the art, and reflects the ratio between the hydrophilic part and the lipophilic part in the molecule.

If two or more first polyglyceryl fatty acid esters are used, the HLB value is determined by a weighted average of the HLB values of all the first polyglyceryl fatty acid esters.

The first polyglyceryl fatty acid ester may be chosen from mono, di, tri and more esters of saturated or unsaturated fatty acid(s).

It is preferable that the first polyglyceryl fatty acid ester comprises 2 to 4 glycerol units, preferably 3 or 4 glycerol units, and more preferably 4 glycerol units.

The fatty acid for the fatty acid moiety or the fatty acid moiety of the first polyglyceryl fatty acid ester may comprise 12 or fewer carbon atoms, preferably 11 or fewer carbon atoms, and more preferably 10 or fewer carbon atoms. The fatty acid for the fatty acid moiety or the fatty acid moiety of the first polyglyceryl fatty acid ester may comprise 4 or more carbon atoms, preferably 6 or more carbon atoms, and more preferably 8 or more carbon atoms. The fatty acid for the fatty acid moiety or the fatty acid moiety of the first polyglyceryl fatty acid ester may have carbon atoms of from 4 to 12, preferably from 6 to 11, and more preferably from 8 to 10 carbon atoms.

The fatty acid for the fatty acid moiety of the first polyglyceryl fatty acid ester may be saturated or unsaturated, and may be selected from caprylic acid, capric acid, and lauric acid.

The first polyglyceryl fatty acid ester(s) may be selected from the group consisting of PG3 caprate (HLB: about 14), PG4 caprylate (HLB: 14), PG4 laurate (HLB: about 14), PG4 caprate (HLB: 14), PG5 myristate (HLB: 15.4), PG5 stearate (HLB: 15), PG6 caprylate (HLB: 14.6), PG6 caprate (HLB: 13.1), PG6 laurate (HLB: 14.1), PG10 laurate (HLB: 15.2), PG10 myristate (HLB: 14.9), PG10 stearate (HLB: 14.1), PG10 isostearate (HLB: 13.7), PG10 oleate (HLB: 13.0), PG10 cocoate (HLB: 16), and mixtures thereof.

It may be preferable that the first polyglyceryl fatty acid ester(s) be selected from the group consisting of PG3 caprate (HLB: about 14), PG4 caprylate (HLB: 14), PG4 laurate (HLB: about 14), PG4 caprate (HLB: 14), and mixtures thereof.

The amount of the first polyglyceryl fatty acid ester(s) in the composition according to the present invention may be 0.01% by weight or more, preferably 0.05% by weight or more, and more preferably 0.1 % by weight or more, relative to the total weight of the composition.

On the other hand, the amount of the first polyglyceryl fatty acid ester(s) in the composition according to the

present invention may be 15% by weight or less, preferably 10% by weight or less, and more preferably 5% by weight or less, relative to the total weight of the composition, with the proviso that the amount of the first polyglyceryl fatty acid ester is not zero.

The amount of the first polyglyceryl fatty acid ester(s) in the composition according to the present invention may range from 0.01% to 15% by weight, preferably from 0.05% to 10% by weight, and more preferably from 0.1% to 5% by weight, relative to the total weight of the composition.

(Second Polyglyceryl Fatty Acid Ester)

The composition according to the present invention may comprise at least one second polyglyceryl fatty acid ester having an HLB value of 10.0 or less, preferably 9.5 or less, and more preferably 9.0 or less. A single type of second polyglyceryl fatty acid ester may be used, but two or more different types of second polyglyceryl fatty acid ester may be used in combination.

The second polyglyceryl fatty acid ester can function as a surfactant, in particular a nonionic surfactant.

The second polyglyceryl fatty acid may have an HLB value of 5.0 to 10.0, preferably 6.0 to 9.5, and more preferably 7.0 to 9.0.

If two or more second polyglyceryl fatty acid esters are used, the HLB value is determined by a weighted average of the HLB values of all the second polyglyceryl fatty acid esters.

The second polyglyceryl fatty acid ester may be chosen from mono, di, tri and more esters of saturated or unsaturated fatty acid(s).

It is preferable that the second polyglyceryl fatty acid ester comprises 2 to 4 glycerol units, preferably 2 or 3 glycerol units, and more preferably 2 glycerol units.

The fatty acid for the fatty acid moiety or the fatty acid moiety of the second polyglyceryl fatty acid ester may comprise 14 or more carbon atoms, preferably 16 or more carbon atoms, and more preferably 18 or more carbon atoms. The fatty acid for the fatty acid moiety or the fatty acid moiety of the second polyglyceryl fatty acid ester may comprise 30 or fewer carbon atoms, preferably 24 or fewer carbon atoms, and more preferably 20 or fewer carbon atoms. The fatty acid for the fatty acid moiety or the fatty acid moiety of the second polyglyceryl fatty acid ester may have from 14 to 30, preferably from 16 to 24, and more preferably from 18 to 20 carbon atoms.

The fatty acid for the fatty acid moiety of the second polyglyceryl fatty acid ester may be saturated or unsaturated, and may be selected from myristic acid, stearic acid, isostearic acid, and oleic acid.

The second polyglyceryl fatty acid ester(s) may be selected from the group consisting of PG2 stearate (HLB: 5.0), PG2 distearate (HLB: 4), PG2 isostearate (HLB: 8), PG2 diisostearate (HLB: 3.2), PG2 triisostearate (HLB: 3), PG2 sesquiisostearate (HLB: about 4), PG2 oleate (HLB: 8), PG2 sesquioleate (HLB: 5.3), PG3 distearate (HLB: 5), PG3 diisostearate (HLB: 5), PG3 dicocoate (HLB: 7), PG5 hexastearate (HLB: 4.0), PG5 trioleate (HLB: 7.0), PG10 pentaoleate (HLB: 6.4), PG2 sesquicaprylate (HLB: about 8), PG2 caprate (HLB: 9.5), PG2 laurate (HLB: 8.5), PG2 myristate (HLB: 10), PG2 isopalmitate (HLB: 9), PG4 oleate (HLB: 10), PG4 stearate (HLB: 9), PG4 isostearate (HLB: 8.2), PG6 distearate (HLB: 8), PG10 distearate (HLB: about 9), PG10 tristearate (HLB: 8), PG10 diisostearate (HLB: 10), PG10 triisostearate (HLB: 8), PG10 tricocoate (HLB: 9), and mixtures thereof.

It may be preferable that the second polyglyceryl fatty acid be selected from the group consisting of PG2 stearate (HLB: 5.0), PG2 distearate (HLB: 4), PG2 isostearate (HLB: 8), PG2 diisostearate (HLB: 3.2), PG2 triisostearate (HLB: 3), PG2 sesquiisostearate (HLB: about 4), PG2 oleate (HLB: 8), PG2 sesquioleate (HLB: 5.3), PG3 distearate (HLB: 5), PG3 diisostearate (HLB: 5), PG3 dicocoate (HLB: 7), PG2 sesquicaprylate (HLB: about 8), PG2 caprate (HLB: 9.5), PG2 laurate (HLB: 8.5), PG2 myristate (HLB: 10), PG2 isopalmitate (HLB: 9), PG4 oleate (HLB: 10), PG4 stearate (HLB: 9), PG4 isostearate (HLB: 8.2), and mixtures thereof.

The amount of the second polyglyceryl fatty acid ester(s) in the composition according to the present invention may be 0.01% by weight or more, preferably 0.05% by weight or more, and more preferably 0.1% by weight or more, relative to the total weight of the composition.

On the other hand, the amount of the second polyglyceryl fatty acid ester(s) in the composition according to the present invention may be 10% by weight or less, preferably 5% by weight or less, and more preferably 1% by weight or less, relative to the total weight of the composition, with the proviso that the amount of the second polyglyceryl fatty acid ester is not zero.

The amount of the second polyglyceryl fatty acid ester(s) in the composition according to the present invention may range from 0.01% to 10% by weight, preferably from 0.05% to 5% by weight, more preferably from 0.1 % to 1 % by weight, relative to the total weight of the composition.

(Weight Ratio Regarding Polyglyceryl Fatty Acid Esters)

According to the present invention, the weight ratio of (the total amounts of the first polyglyceryl fatty acid

ester(s) and the second polyglyceryl fatty acid ester(s))/the amount of the oil(s) may be 4.0 or less, preferably 3.0 or less, and more preferably 2.0 or less.

Conventionally, the weight ratio of the amount(s) of glyceryl fatty acid ester(s)/the oil(s) is much higher, such as 6.0.

Therefore, the composition according to the present invention can reduce or limit the total amounts of the polyglyceryl fatty acid esters.

Since the present invention can reduce the total amounts of the polyglyceryl fatty acid esters, the composition according to the present invention can provide no sticky feeling or can provide a further reduced sticky feeling to the touch.

It may be preferable that the weight ratio of (the total amounts of the first polyglyceryl fatty acid ester(s) and the second polyglyceryl fatty acid ester(s))/the amount of the oil(s) be more than 0.5, preferably more than 1.0, and more preferably more than 1.5.

On the other hand, according to the present invention, the weight ratio of the amount of the first polyglyceryl fatty acid ester(s)/the second polyglyceryl fatty acid ester(s) may be 1 or more, preferably 1.5 or more, and more preferably 2 or more; may be 5 or less, preferably 4 or less, and more preferably 3 or less; and may be from 1 to 5, preferably from 1.5 to 4, and more preferably from 2 to 3.

(Average HLB of Polyglyceryl Fatty Acid Esters)

The average HLB value of the first polyglyceryl fatty acid ester(s) and the second polyglyceryl fatty acid ester(s) can be calculated as a weighted average of all the first and second polyglyceryl fatty acid esters.

The average HLB of the first polyglyceryl fatty acid ester(s) and the second polyglyceryl fatty acid ester(s) in the composition according to the present invention may be 11.0 or more, preferably 11.5 or more, and more preferably 12.0 or more.

The average HLB of the first polyglyceryl fatty acid ester(s) and the second polyglyceryl fatty acid ester(s) in the composition according to the present invention may be 14.0 or less, preferably 13.5 or less, and more preferably 13.0 or less.

Thus, in one embodiment of the present invention, the average HLB of the first polyglyceryl fatty acid ester(s) and the second polyglyceryl fatty acid ester(s) in the composition according to the present invention may range from 11.0 to 14.0, preferably from 11.5 to 13.5, and more preferably from 12.0 to 13.0.

[Polyol]

The composition according to the present invention may further comprise at least one polyol. A single type of polyol may be used, but two or more different types of polyol may be used in combination.

The term "polyol" here means an alcohol having two or more hydroxy groups, and does not encompass a saccharide or a derivative thereof. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acyl group or a carbonyl group.

The polyol may be a $C_2$-$C_{12}$ polyol, preferably a $C_2$-$C_9$ polyol, comprising at least 2 hydroxy groups, and preferably 2 to 5 hydroxy groups.

The polyol may be a natural or synthetic polyol. The polyol may have a linear, branched or cyclic molecular structure.

The polyol may be selected from glycerins and derivatives thereof, and glycols and derivatives thereof. The polyol may be selected from the group consisting of glycerin, diglycerin, polyglycerin, ethyleneglycol, diethyleneglycol, propyleneglycol, dipropyleneglycol, butyleneglycol, pentyleneglycol, hexyleneglycol, 1,3-propanediol, 1,5-pentanediol, caprylyl glycol, polyethyleneglycol (5 to 50 ethyleneoxide groups), and sugars such as sorbitol.

The amount of the polyol(s) in the composition according to the present invention may be 0.01% by weight or more, preferably 0.05% by weight or more, and more preferably 0.1% by weight or more, relative to the total weight of the composition.

On the other hand, the amount of the polyol(s) in the composition according to the present invention may be 25% by weight or less, preferably 20% by weight or less, and more preferably 15% by weight or less, relative to the total weight of the composition, with the proviso that the amount of the polyol is not zero.

Thus, the polyol(s) may be present in the composition according to the present invention in an amount ranging from 0.01% to 25% by weight, and preferably from 0.05% to 20% by weight, such as from 0.1 % to 15% by weight, relative to the total weight of the composition.

[Other Ingredients]

The composition according to the present invention may further comprise one or more monoalcohols which are in the form of a liquid at room temperature (25°C), such as for example linear or branched monoalcohols comprising from 1 to 6 carbon atoms, such as ethanol, propanol, butanol, isopropanol, isobutanol, pentanol, and hexanol.

The amount of the monoalcohol(s) in the composition according to the present invention may be 0.01% by weight or more, preferably 0.1% by weight or more, and more preferably 1% by weight or more, relative to the total weight of the composition.

On the other hand, the amount of the monoalcohol(s) in the composition according to the present invention may be 15% by weight or less, preferably 10% by weight or less, and more preferably 5% by weight or less, relative to the total weight of the composition.

Thus, the amount of the monoalcohol(s) in the composition according to the present invention may range from 0.01% to 15% by weight, preferably from 0.1% to 10% by weight, and more preferably from 1% to 5% by weight, relative to the total weight of the composition.

The composition according to the present invention may also include various adjuvants conventionally used in cosmetic and dermatological compositions, such as thickeners, anionic, non-ionic, cationic, and amphoteric or zwitterionic polymers, additional antioxidants, coloring agents, chelating agents, sequestering agents, fragrances, dispersing agents, conditioning agents, film-forming agents, preservatives, co-preservatives, and mixtures thereof, except for the ingredients as explained above.

The composition according to the present invention may further include baicalin. If the composition according to the present invention includes baicalin, it may be preferable that the amount of the baicalin is limited to be 0.5% by weight or less relative to the total weight of the composition. The composition according to the present invention may include no baicalin.

In one embodiment, the composition according to the present invention may be free from any UV filter, because it is stable against light. The term "free from" here means that the composition according to the present invention may contain a limited amount of at least one UV filter. However, it is preferable that the amount of the UV filter be limited such that it is less than 1% by weight, more preferably less than 0.1% by weight, and even more preferably less than 0.01% by weight, relative to the total weight of the composition. It may be most preferable that the composition according to the present invention comprises no UV filter.

In another embodiment, the composition according to the present invention may be free from a polyoxyethylene-based nonionic surfactant. The term "free from" here means that the composition according to the present invention may contain a limited amount of a polyoxyethylene-based nonionic surfactant. However, it is preferable that the amount of the polyoxyethylene-based nonionic surfactant be limited such that it is less than 1% by weight, more preferably less than 0.1% by weight, and even more preferably less than 0.01% by weight, relative to the total weight of the composition. It may be most preferable that the composition according to the present invention comprises no polyoxyethylene-based nonionic surfactant.

(Preparation)

The composition according to the present invention can be prepared by mixing the essential ingredient(s) as explained above, and optional ingredient(s), if necessary, as explained above.

The (a) polydatin may be derived from plants. Therefore, as the (a) polydatin, for example, a plant extract including the (a) polydatin may be used. For example, as the (a) polydatin, Polygonium Cuspidatum Root Extract marketed by Guilin Layn Natural Ingredients may be used.

The (b) caffeoyl quinic acid compound may be derived from plants. Therefore, as the (b) caffeoyl quinic acid compound, for example, a plant extract including the (b) caffeoyl quinic acid compound may be used. For example, as the (b) caffeoyl quinic acid compound, Eucommia Leaves Extract Chlorogenic Acid 98% marketed by Guilin Layn Natural Ingredients may be used.

Therefore, the composition according to the present invention can be prepared by mixing, for example, the (a) polydatin and/or a first plant extract including the (a) polydatin and the (b) caffeoyl quinic acid compound and/or a second plant extract including the (b) caffeoyl quinic acid compound, as well as the above ingredients (c) and (d), and optional ingredient(s), if necessary, as explained above.

The method and means to mix the above essential and optional ingredients are not limited. Any conventional method and means can be used to mix the above essential and optional ingredients to prepare the composition according to the present invention.

The composition according to the present invention may be prepared without a large amount of energy such as required by a homogenizer. Thus, the composition according to the present invention may be prepared by using a small amount of energy such as gently stirring the ingredients of the composition. Therefore, the composition according

to the present invention is environmentally friendly in view of the preparation approach thereof.

[Form]

The composition according to the present invention is not limited. For example, the composition according to the present invention may be in the form of an aqueous solution.

If the composition according to the present invention comprises at least one oil, it is preferable that the composition according to the present invention be in the form of an O/W emulsion which comprises oil phases dispersed in a continuous aqueous phase. The dispersed oil phases can be oil droplets in the aqueous phase. In this case, it is preferable that the composition according to the present invention comprises at least one emulsifier as explained above.

The O/W architecture or structure, which consists of oil phases dispersed in an aqueous phase, has an external aqueous phase, and therefore if the composition according to the present invention has the O/W architecture or structure, it can provide a pleasant feeling during use because of the feeling of immediate freshness that the aqueous phase can provide.

The composition according to the present invention may be in the form of a nano- or micro-emulsion.

The "micro-emulsion" may be defined in two ways, namely, in a broad sense and in a narrow sense. That is to say, there is the one case ("micro-emulsion in the narrow sense") in which the micro-emulsion refers to a thermodynamically stable isotropic single liquid phase containing a ternary system having three ingredients of an oily component, an aqueous component and a surfactant, and there is the second case ("micro-emulsion in the broad sense") in which among thermodynamically unstable typical emulsion systems the micro-emulsion additionally includes those such emulsions presenting transparent or translucent appearances due to their smaller particle sizes (Satoshi Tomomasa, et al., Oil Chemistry, Vol. 37, No. 11 (1988), pp. 48-53). The "micro-emulsion" as used herein refers to a "micro-emulsion in the narrow sense", i.e., a thermodynamically stable isotropic single liquid phase.

The micro-emulsion refers to either one state of an O/W (oil-in-water) type microemulsion in which oil is solubilized by micelles, a W/O (water-in-oil) type microemulsion in which water is solubilized by reverse micelles, or a bicontinuous microemulsion in which the number of associations of surfactant molecules are rendered infinite so that both the aqueous phase and oil phase have a continuous structure.

The micro-emulsion may have a dispersed phase with a particle size of 100 nm or less, preferably 50 nm or less, and more preferably 20 nm or less, measured by laser granulometry.

The "nano-emulsion" here means an emulsion characterized by a dispersed phase with a size of less than 350 nm, the dispersed phase being stabilized by a crown of the (b) and (c) nonionic surfactants that may optionally form a liquid crystal phase of lamellar type, at the dispersed phase/continuous phase interface. In the absence of specific opacifiers, the transparency of the nano-emulsions arises from the small size of the dispersed phase, this small size being obtained by virtue of the use of mechanical energy.

Nanoemulsions can be distinguished from microemulsions by their structure. Specifically, micro-emulsions are thermodynamically stable dispersions formed from, for example, micelles which are formed by emulsifiers and swollen with oil. Furthermore, microemulsions do not require substantial mechanical energy in order to be prepared.

The nano-emulsion may have a dispersed phase with a particle size of 300 nm or less, preferably 200 nm or less, and more preferably 100 nm or less, measured by laser granulometry.

It is even more preferable that the particle size of the (a) oil be 35 nm or less, preferably 30 nm or less, and more preferably 25 nm or less, if the composition according to the present invention is in the form of an O/W emulsion. The particle size can be measured by a dynamic light scattering method. The particle size measurement can be performed by, for example, the Particle Size Analyzer ELSZ-2000 series, marketed by Otsuka Electronics Co., Ltd.

The particle size can be a volume-average particle diameter or a number-average particle diameter, preferably a volume-average particle diameter.

The composition according to the present invention can be transparent.

The transparency may be measured by measuring turbidity. For example, turbidity can be measured with a 2100Q (marketed by Hach Company) having a round cell (25 mm in diameter and 60 mm height) and a tungsten filament lamp which can emit visible light (between 400 and 800 nm, preferably from 400 to 500 nm). The measurement can be performed on the undiluted composition. The blank may be determined with distilled water.

The composition according to the present invention has a turbidity of 150 NTU or less, preferably 130 NTU or less and more preferably 110 NTU or less.

It may be more preferable that the composition according to the present invention is in the form of a nano- or micro- O/W emulsion.

It may be even more preferable that the composition according to the present invention is in the form of a nano- or micro- O/W gel emulsion. For example, the aqueous phase of the nano- or micro- O/W gel emulsion may include at least one thickener such as xanthan gum.

[Process and Use]

It is preferable that the composition according to the present invention be a cosmetic or dermatological composition, preferably a cosmetic composition, and more preferably a skin cosmetic composition. The composition according to the present invention may be in the form of a lotion, a cream, a serum, and the like.
The composition according to the present invention can be used for a non-therapeutic process, such as a cosmetic process, for treating a keratin substance such as skin, in particular face, by being applied to the keratin substance.
Thus, the present invention also relates to a cosmetic process for treating a keratin substance such as skin, comprising the step of applying the composition according to the present invention to the keratin substance.
The present invention may also relate to a use of the composition according to the present invention as or in a cosmetic product such as skin care products. The skin care product may be a lotion, a cream, a serum, and the like.
In other words, the composition according to the present invention can be used, as it is, as a cosmetic product. Alternatively, the composition according to the present invention can be used as an element of a cosmetic product. For example, the composition according to the present invention can be added to or combined with any other elements to form a cosmetic product.
Another aspect of the present invention may relate to a use of

(b) at least one caffeoyl quinic acid compound; and
(c) at least one Vitamin B3 compound,
for stabilizing, in particular against light,
(a) polydatin
in a composition comprising (d) water.

Another aspect of the present invention may also relate to a use of:

(b) at least one caffeoyl quinic acid compound; and
(c) at least one Vitamin B3 compound,
as a stabilizer, in particular against light, of
(a) polydatin
in a composition comprising (d) water.

The above explanations regarding the ingredients (a) to (d), as well as the optional ingredients, for the composition according to the present invention can apply to those for the uses and processes according to the present invention. The explanations regarding the preparation and forms of the composition according to the present invention can also apply to those of the composition recited in the above uses and processes.

EXAMPLES

The present invention will be described in more detail by way of examples which however should not be construed as limiting the scope of the present invention.

[Examples 1-3 and Comparative Examples 1-5]

The following compositions in the form of a micro-emulsion according to Examples 1-3 and Comparative Examples 1-5 shown in Table 1, were prepared by mixing the ingredients shown in Table 1 as follows. The numerical values for the amounts of the ingredients shown in Table 1 are all based on "% by weight" as active raw materials.

Table 1

| Ingredients | Ex. 1 | Ex. 2 | Ex. 3 | Comp. Ex. 1 | Comp. Ex. 2 | Comp. Ex. 3 | Comp. Ex. 4 | Comp. Ex. 5 |
|---|---|---|---|---|---|---|---|---|
| Propylene Glycol | 3 | 3 | 3 | 3 | 3 | 3 | 3 | 3 |
| Pentylene Glycol | 2 | 2 | 2 | 2 | 2 | 2 | 2 | 2 |
| Glycerin | 8.8 | 8.8 | 8.8 | 8.8 | 8.8 | 8.8 | 8.8 | 8.8 |

(continued)

| Ingredients | Ex. 1 | Ex. 2 | Ex. 3 | Comp. Ex. 1 | Comp. Ex. 2 | Comp. Ex. 3 | Comp. Ex. 4 | Comp. Ex. 5 |
|---|---|---|---|---|---|---|---|---|
| Caprylyl Glycol | 0.3 | 0.3 | 0.3 | 0.3 | 0.3 | 0.3 | 0.3 | 0.3 |
| Arginine | 1.205 | 1.205 | 1.205 | 1.205 | 1.205 | 1.205 | 1.205 | 1.205 |
| Water | qsp 100 | qsp 100 | qsp 100 | qsp 100 | qsp 100 | qsp 100 | qsp 100 | qsp 100 |
| Caramel | 0.05 | 0.05 | 0.05 | 0.05 | 0.05 | 0.05 | 0.05 | 0.05 |
| Sodium Lauroyl Glutamate | 0.05 | 0.05 | 0.05 | 0.05 | 0.05 | 0.05 | 0.05 | 0.05 |
| Citric Acid | 0.165 | 0.165 | 0.165 | 0.165 | 0.165 | 0.165 | 0.165 | 0.165 |
| Caffeine | 1 | 1 | 1 | 1 | 1 | 1 | 1 | 1 |
| Niacinamide | 4 | 4 | 4 | 4 | 4 | 4 | 4 | - |
| Baicalin | - | - | - | - | 1.0 | 1.5 | - | - |
| Chlorogenic Acid | 1.5 | 1.0 | 0.5 | 0.25 | - | - | - | - |
| Polydatin | 0.5 | 0.5 | 0.5 | 0.5 | 0.5 | 0.5 | 0.5 | 0.5 |
| Salicylic Acid | 0.2 | 0.2 | 0.2 | 0.2 | 0.2 | 0.2 | 0.2 | 0.2 |
| Magnesium Gluconate | 0.5 | 0.5 | 0.5 | 0.5 | 0.5 | 0.5 | 0.5 | 0.5 |
| Isopropyl Myristate | 0.8 | 0.8 | 0.8 | 0.8 | 0.8 | 0.8 | 0.8 | 0.8 |
| Polyglyceryl-2 Oleate | 0.45 | 0.45 | 0.45 | 0.45 | 0.45 | 0.45 | 0.45 | 0.45 |
| Polyglyceryl-4 Caprate | 1.05 | 1.05 | 1.05 | 1.05 | 1.05 | 1.05 | 1.05 | 1.05 |
| Glycerin | 1.2 | 1.2 | 1.2 | 1.2 | 1.2 | 1.2 | 1.2 | 1.2 |
| Fragrance | 0.01 | 0.01 | 0.01 | 0.01 | 0.01 | 0.01 | 0.01 | 0.01 |
| Xanthan Gum | 0.3 | 0.3 | 0.3 | 0.3 | 0.3 | 0.3 | 0.3 | 0.3 |
| Ethanol | 2 | 2 | 2 | 2 | 2 | 2 | 2 | 2 |
| Thermal Stability | Good | Good | Good | Good | Poor | Poor | Good | Good |
| Photo Stability | 65% | 63% | 53% | 30% | NA | NA | 36% | 9% |

[Evaluations]

(Thermal Stability)

Each of the compositions according to Examples 1-3 and Comparative Examples 1-5 was charged into a transparent glass bottle, and the glass bottle was stored under the temperature of 4°C for 1 month. 1 month later, the aspect of the glass bottle was visually observed and evaluated in accordance with the following criteria.

Good: No crystal particle was observed
Poor: Crystal particle was observed

The results are shown in the line of "Thermal Stability" in Table 1.

The compositions according to Examples 1-3 as well as Comparative Examples 1, 4 and 5 were stable for a long period of time even under the cold temperature, such that no crystal particle was formed.

The compositions according to Comparative Examples 2 and 3 were unstable, such that crystal particle was formed under the cold temperature. Thus, it was found that the use of baicalin in an amount of 1.0% by weight or more is not preferable to prepare a thermally stable composition.

(Photo Stability)

Each of the compositions according to Examples 1-3 and Comparative Examples 1, 4 and 5 was spread on two glass plates with the amount of $2mg/cm^2$. One of the two glass plates was placed under Xenon light, while the other was placed under no light. They were left for 30 minutes at a temperature of 25°C. Then, the two glass plates were immersed into a 25g of methanol and stirred with a stirrer tip for 30 minutes to extract polydatin. The methanol solution was measured by HPLC (reverse phase) to quantify polydatin. By the equation below, the photostability of polydatin was calculated:

$$\text{Photostability (\%)} = \text{(polydatin(\%) with Xe light)/(polydatin(\%) without Xe light)}$$

The results are shown in the line of "Photo Stability" in Table 1. The "NA" in Table 1 means Not Available.

The compositions according to Examples 1-3 were more stable under light such that 50% of polydatin remained even after the exposure to light.

The compositions according to Comparative Examples 1, 4 and 5 were less stable under light such that the photostability thereof was less than 50%.

## Claims

1. A composition, comprising:

   (a) polydatin;
   (b) at least one caffeoyl quinic acid compound;
   (c) at least one Vitamin B3 compound; and
   (d) water,

   wherein
   the amount of the (b) caffeoyl quinic acid compound is 0.5% by weight or more, preferably 1.0% by weight or more, and more preferably 1.5% by weight or more, relative to the total weight of the composition.

2. The composition according to Claim 1, wherein the (a) polydatin is derived from plants.

3. The composition according to Claim 1 or 2, wherein the amount of the (a) polydatin in the composition ranges from 0.01% to 3% by weight, preferably from 0.05% to 2% by weight, and more preferably from 0.1 % to 1 % by weight, relative to the total weight of the composition.

4. The composition according to any one of Claims 1 to 3, wherein the (b) caffeoyl quinic acid compound is represented by the following formula (I):

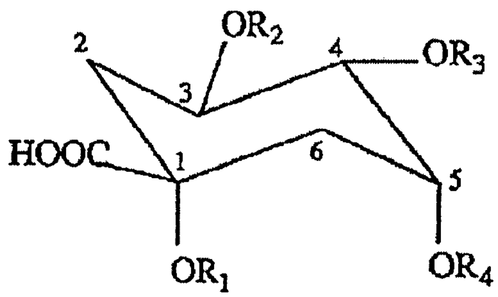

(I)

in which $R_1$, $R_2$, $R_3$, and $R_4$ independently represents a hydrogen atom or the caffeoyl radical represented by the following formula (II):

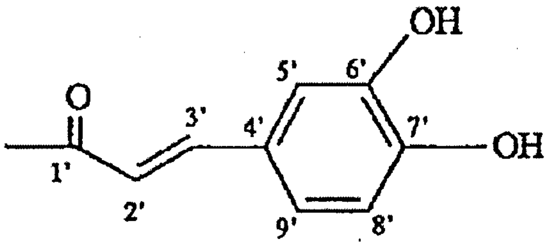

(II)

provided that at least one of $R_1$, $R_2$, $R_3$, and $R_4$ represents the caffeoyl radical of the formula (II).

**5.** The composition according to any one of Claims 1 to 4, wherein the (b) caffeoyl quinic acid compound is chlorogenic acid.

**6.** The composition according to any one of Claims 1 to 5, wherein the (b) caffeoyl quinic acid compound is derived from plants.

**7.** The composition according to any one of Claims 1 to 6, wherein the amount of the (b) caffeoyl quinic acid compound(s) in the composition ranges from 0.5% to 15% by weight, preferably from 1.0% to 10% by weight, and more preferably from 1.5% to 5% by weight, relative to the total weight of the composition.

**8.** The composition according to any one of Claims 1 to 7, wherein the (c) Vitamin B3 compound is represented by the following formula (III):

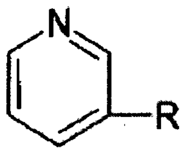

(III)

in which R denotes $-CONH_2$, $-COOH$, $CH_2OH$, $-CO-NH-CH_2-COOH$ or $-CO-NH-OH$.

**9.** The composition according to any one of Claims 1 to 8, wherein the (c) Vitamin B3 compound is niacinamide.

**10.** The composition according to any one of Claims 1 to 9, wherein the amount of the (c) Vitamin B3 compound(s) in the composition ranges from 0.1% to 15% by weight, preferably from 0.5% to 10% by weight, and more preferably from 1% to 5% by weight, relative to the total weight of the composition.

**11.** The composition according to any one of Claims 1 to 10, wherein the weight ratio of the amount of the (a) polydatin/the (b) caffeoyl quinic acid compound(s) is from 0.1 to 1, preferably from 0.2 to 0.5, and more preferably from 0.3 to 0.4.

**12.** The composition according to any one of Claims 1 to 11, wherein the weight ratio of the amount of the (a) polydatin/the (c) Vitamin B3 compound(s) is from 0.01 to 0.5, preferably from 0.05 to 0.3, and more preferably from 0.1 to 0.2.

**13.** The composition according to any one of Claims 1 to 12, wherein the weight ratio of the amount of the (c) Vitamin B3 compound(s)/the (b) caffeoyl quinic acid compound(s) is from 1 to 8, preferably from 1.5 to 4, and more preferably from 2 to 3.

**14.** The composition according to any one of Claims 1 to 13, wherein the composition is in the form of an O/W emulsion, preferably a nano- or micro- O/W emulsion, and more preferably a nano- or micro- O/W gel emulsion.

**15.** A cosmetic process for treating a keratin substance such as skin, comprising the step of applying the composition according to any one of Claims 1 to 14 to the keratin substance.

**Patentansprüche**

1. Zusammensetzung, die Folgendes umfasst:

   (a) Polydatin;
   (b) mindestens eine Caffeoylchinasäure-Verbindung;
   (c) mindestens eine Vitamin B3-Verbindung; und
   (d) Wasser,

   wobei
   die Menge der (b) Caffeoylchinasäure-Verbindung 0,5 Gew.-% oder mehr, vorzugsweise 1,0 Gew.-% oder mehr und mehr bevorzugt 1,5 Gew.-% oder mehr beträgt, bezogen auf das Gesamtgewicht der Zusammensetzung.

2. Zusammensetzung nach Anspruch 1, wobei das (a) Polydatin aus Pflanzen gewonnen ist.

3. Zusammensetzung nach Anspruch 1 oder 2, wobei die Menge des (a) Polydatins in der Zusammensetzung im Bereich von 0,01 Gew.-% bis 3 Gew.-%, vorzugsweise von 0,05 Gew.-% bis 2 Gew.-% und mehr bevorzugt von 0,1 Gew.-% bis 1 Gew.-% liegt, bezogen auf das Gesamtgewicht der Zusammensetzung.

4. Zusammensetzung nach einem der Ansprüche 1 bis 3, wobei die (b) Caffeoylchinasäure-Verbindung durch die folgende Formel (I) dargestellt wird:

   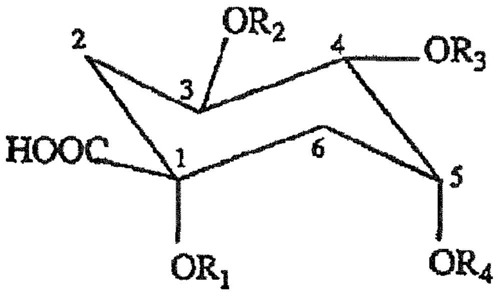

   (I)

   in der $R_1$, $R_2$, $R_3$ und $R_4$ unabhängig voneinander ein Wasserstoffatom oder das Caffeoylradikal repräsentiert, das durch folgende Formel (II) dargestellt wird:

   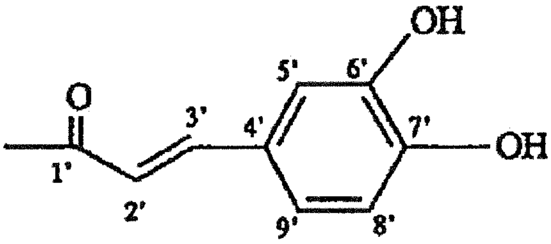

   (II)

   mit der Maßgabe, dass mindestens eines von $R_1$, $R_2$, $R_3$ und $R_4$ das Caffeoylradikal der Formel (II) repräsentiert.

5. Zusammensetzung nach einem der Ansprüche 1 bis 4, wobei es sich bei der (b) Caffeoylchinasäure-Verbindung um Chlorogensäure handelt.

6. Zusammensetzung nach einem der Ansprüche 1 bis 5, wobei die (b) Caffeoylchinasäure-Verbindung aus Pflanzen gewonnen ist.

7. Zusammensetzung nach einem der Ansprüche 1 bis 6, wobei die Menge der (b) Caffeoylchinasäure-Verbindung(en) in der Zusammensetzung im Bereich von 0,5 Gew.-% bis 15 Gew.-%, vorzugsweise von 1,0 Gew.-% bis 10 Gew.-% und mehr bevorzugt von 1,5 Gew.-% bis 5 Gew.-% liegt, bezogen auf das Gesamtgewicht der Zusammensetzung.

8. Zusammensetzung nach einem der Ansprüche 1 bis 7, wobei die (c) Vitamin B3-Verbindung durch die folgende Formel (III) repräsentiert wird:

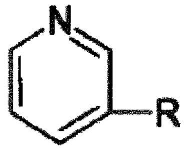

(III)

in der R $-CONH_2$, $-COOH$, $CH_2OH$, $-CO-NH-CH_2-COOH$ oder $-CO-NH-OH$ bezeichnet.

9. Zusammensetzung nach einem der Ansprüche 1 bis 8, wobei es sich bei der (c) Vitamin B3-Verbindung um Niacinamid handelt.

10. Zusammensetzung nach einem der Ansprüche 1 bis 9, wobei die Menge der (c) Vitamin B3-Verbindung(en) in der Zusammensetzung im Bereich von 0,1 Gew.-% bis 15 Gew.-%, vorzugsweise von 0,5 Gew.-% bis 10 Gew.-% und mehr bevorzugt von 1 Gew.-% bis 5 Gew.-% liegt, bezogen auf das Gesamtgewicht der Zusammensetzung.

11. Zusammensetzung nach einem der Ansprüche 1 bis 10, wobei das Gewichtsverhältnis der Menge des (a) Polydatins zu der bzw. den Caffeoylchinasäure-Verbindung(en) von 0,1 bis 1, vorzugsweise von 0,2 bis 0,5 und mehr bevorzugt von 0,3 bis 0,4 beträgt.

12. Zusammensetzung nach einem der Ansprüche 1 bis 11, wobei das Gewichtsverhältnis der Menge des (a) Polydatins zu der bzw. den (c) Vitamin B3-Verbindung(en) von 0,01 bis 0,5, vorzugsweise von 0,05 bis 0,3 und mehr bevorzugt von 0,1 bis 0,2 beträgt.

13. Zusammensetzung nach einem der Ansprüche 1 bis 12, wobei das Gewichtsverhältnis der Menge der (c) Vitamin B3-Verbindung(en) zu der bzw. den (b) Caffeoylchinasäure-Verbindung(en) von 1 bis 8, vorzugsweise von 1,5 bis 4 und mehr bevorzugt von 2 bis 3 beträgt.

14. Zusammensetzung nach einem der Ansprüche 1 bis 13, wobei die Zusammensetzung in Form einer Ö/W-Emulsion, vorzugsweise einer Nano- oder Mikro-ÖIW-Emulsion und mehr bevorzugt einer Nano- oder Mikro-ÖIW-Gel-Emulsion vorliegt.

15. Kosmetisches Verfahren zur Behandlung einer Keratinsubstanz wie zum Beispiel der Haut, mit dem Schritt des Auftragens der Zusammensetzung nach einem der Ansprüche 1 bis 14 auf die Keratinsubstanz.

## Revendications

1. Composition, comprenant :

   (a) de la polydatine ;
   (b) au moins un composé d'acide caféoylquinique ;
   (c) au moins un composé de vitamine B3 ; et
   (d) de l'eau,

   dans laquelle
   la quantité du (b) composé d'acide caféoylquinique est supérieure ou égale à 0,5 % en poids, de préférence supérieure ou égale à 1,0 % en poids, et plus préférentiellement supérieure ou égale à 1,5 % en poids, par rapport au poids total de la composition.

2. Composition selon la revendication 1, dans laquelle la (a) polydatine est issue de plantes.

3. Composition selon la revendication 1 ou 2, dans laquelle la quantité de la (a) polydatine dans la composition va de 0,01 % à 3 % en poids, de préférence de 0,05 % à 2 % en poids, et plus préférentiellement de 0,1 % à 1 % en poids, par rapport au poids total de la composition.

**4.** Composition selon l'une quelconque des revendications 1 à 3, dans laquelle le (b) composé d'acide caféoylquinique est représenté par la formule (I) suivante :

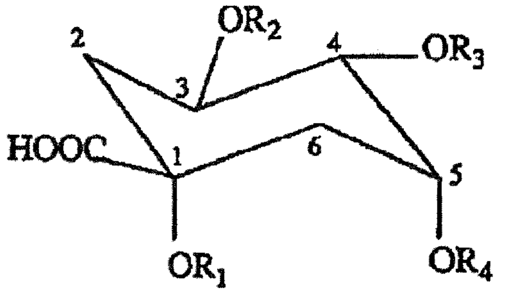

(I)

dans laquelle $R_1$, $R_2$, $R_3$, et $R_4$ représentent indépendamment un atome d'hydrogène ou le radical caféoyle représenté par la formule (II) suivante :

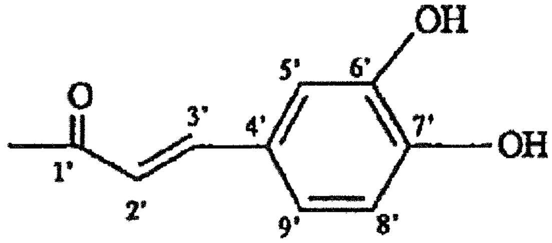

(II)

à condition qu'au moins l'un de $R_1$, $R_2$, $R_3$, et $R_4$ représente le radical caféoyle de la formule (II).

**5.** Composition selon l'une quelconque des revendications 1 à 4, dans laquelle le (b) composé d'acide caféoylquinique est l'acide chlorogénique.

**6.** Composition selon l'une quelconque des revendications 1 à 5, dans laquelle le (b) composé d'acide caféoylquinique est issu de plantes.

**7.** Composition selon l'une quelconque des revendications 1 à 6, dans laquelle la quantité du ou des (b) composés d'acide caféoylquinique dans la composition va de 0,5 % à 15 % en poids, de préférence de 1,0 % à 10 % en poids, et plus préférentiellement de 1,5 % à 5 % en poids, par rapport au poids total de la composition.

**8.** Composition selon l'une quelconque des revendications 1 à 7, dans laquelle le (c) composé de vitamine B3 est représenté par la formule (III) suivante :

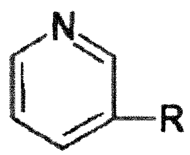

(III)

dans laquelle R désigne $-CONH_2$, $-COOH$, $CH_2OH$, $-CO-NH-CH_2-COOH$ ou $-CO-NH-OH$.

**9.** Composition selon l'une quelconque des revendications 1 à 8, dans laquelle le (c) composé de vitamine B3 est le niacinamide.

**10.** Composition selon l'une quelconque des revendications 1 à 9, dans laquelle la quantité du ou des (c) composés de vitamine B3 dans la composition va de 0,1 % à 15 % en poids, de préférence de 0,5 % à 10 % en poids, et plus préférentiellement de 1 % à 5 % en poids, par rapport au poids total de la composition.

**11.** Composition selon l'une quelconque des revendications 1 à 10, dans laquelle le rapport pondéral de

la quantité de la (a) polydactine/du ou des (b) composés d'acide caféoylquinique
est de 0,1 à 1, de préférence de 0,2 à 0,5, et plus préférentiellement de 0,3 à 0,4.

**12.** Composition selon l'une quelconque des revendications 1 à 11, dans laquelle le rapport pondéral de

la quantité la (a) polydactine/du ou des (b) composés de vitamine B3
est de 0,01 à 0,5, de préférence de 0,05 à 0,3, et plus préférentiellement de 0,1 à 0,2.

**13.** Composition selon l'une quelconque des revendications 1 à 12, dans laquelle le rapport pondéral de

la quantité du ou des (c) composés de vitamine B3/du ou des (b) composés d'acide caféoylquinique
est de 1 à 8, de préférence de 1,5 à 4, et plus préférentiellement de 2 à 3.

**14.** Composition selon l'une quelconque des revendications 1 à 13, dans laquelle la composition se présente sous forme d'une émulsion H/E, de préférence une nano ou microémulsion H/E, et plus préférentiellement une nano ou microémulsion gel H/E.

**15.** Procédé cosmétique de traitement d'une substance kératineuse telle que la peau, comprenant l'étape d'application de la composition selon l'une quelconque des revendications 1 à 14 sur la substance kératineuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LEE J. et al.** Hydrotropic Solubilization of Paclitaxel: Analysis of Chemical Structures for Hydrotropic Property. *Pharmaceutical Research,* 2003, vol. 20 (7
- **LEE S. et al.** Hydrotropic Polymers: Synthesis and Characterization of Polymers Containing Picolylnicotinamide Moieties. *Macromolecules,* 2003, vol. 36, 2248-2255
- Walter Noll's Chemistry and Technology of Silicones. Academic Press, 1968
- **TODD ; BYERS.** Volatile Silicone Fluids for Cosmetics. *Cosmetics and Toiletries,* vol. 91 (76), 27-32
- **SATOSHI TOMOMASA et al.** *Oil Chemistry,* 1988, vol. 37 (11), 48-53